# EUROPÄISCHE PATENTSCHRIFT

(11) **EP 1 915 087 B1**
(45) Veröffentlichungstag und Bekanntmachung des Hinweises auf die Patenterteilung: **05.10.2011**
(21) Anmeldenummer: 05782840.2
(22) Anmeldetag: 17.08.2005
(51) Int. Cl.: A61B 1/00

(54) **HYGIENESCHUTZ FÜR ENDOSKOPE UND ENDOSKOP MIT HYGIENESCHUTZ**
HYGIENE PROTECTOR FOR ENDOSCOPES AND ENDOSCOPE WITH HYGIENE PROTECTOR
PROTECTION HYGIENIQUE DESTINEE A DES ENDOSCOPES ET ENDOSCOPE COMPORTANT UNE PROTECTION HYGIENIQUE

(43) Veröffentlichungstag der Anmeldung: 30.04.2008
(73) Patentinhaber: Kress, Jürgen, 84051 Essenbach (DE)
(72) Erfinder: BRAUN, Michael, 71522 Backnang (DE); GEIS, John, S., 26160 Bad Zwischenahn (DE)
(74) Vertreter: Cullinane, Marietta Bettina
(86) Internationale Anmeldenummer: PCT/DE2005/001450
(87) Internationale Veröffentlichungsnummer: WO 2007/019811

(56) Entgegenhaltungen:
- WO-A-94/22358
- WO-A-2004/060149
- WO-A-2005/037087
- US-A- 4 281 646
- US-A- 4 741 326
- US-A- 5 419 311

## Beschreibung

Die vorliegende Erfindung betrifft einen Hygieneschutz für ein Endoskop, ein Endoskop mit Hygieneschutz sowie ein Verfahren zum Aufbringen eines Hygieneschutzes auf ein Endoskop.

In der Endoskopie können unter Einsatz von Endoskopen sowohl Untersuchungen zu Diagnose-Zwecken als auch kleinere operative Eingriffe vorgenommen werden. Für therapeutische Verwendungen ist in dem Endoskop neben der Optik auch ein Arbeitskanal vorgesehen, über den beispielsweise Biopsieproben entnommen werden können. Da Endoskope bei der Behandlung in Körperhöhlen des menschlichen Körpers eingeführt werden, müssen diese gut gereinigt werden. Je vielseitiger das Endoskop verwendet werden kann, umso komplexer wird dessen Aufbau, wodurch sich die Reinigung des Endoskops erschwert. Insbesondere ist es bei Endoskopen, die optische Elemente, wie beispielsweise einen Lichtleiter und Leuchten zur Ausleuchtung des zu untersuchenden Bereichs, aufweisen, notwendig einen Spülkanal vorzusehen, über den die optischen Elemente während des Einsatzes durch Luft und/oder Wasser gereinigt werden können. Da dieser Kanal mit den Körperflüssigkeiten in Kontakt kommt, muss auch dieser nach dem Gebrauch des Endoskops gereinigt werden. Ebenso steht ein so genannter Arbeitskanal, über den beispielsweise Biopsieproben und überflüssiges Sekret sowie Gewebereste entnommen werden können, mit den Körperflüssigkeiten in Kontakt, so dass auch dieser nach jedem Gebrauch einer intensiven Reinigung zu unterziehen ist.

Diese Kanäle sind aufgrund ihrer Anordnung in dem Endoskop, insbesondere in dem Endoskopschaft schwer zugänglich und müssen somit einer aufwendigen Reinigung unterzogen werden.

Um diesem Problem Rechnung zu tragen wird im Stand der Technik die Verwendung von Schutzhüllen für Endoskope vorgeschlagen. In der WO 2004 060 149 A1 wird beispielsweise ein Hygieneschutz für Endoskope vorgeschlagen, der aus einer Hülle, die am distalen Ende geschlossen sowie zumindest stirnseitig für optische Informationen durchlässig ist und in axialer Richtung des Endoskops kondomartig aufrollbar ist, besteht. Der Hygieneschutz weist zudem einen oder mehrere parallel zum Endoskop verlaufende Funktionskanäle auf, die am distalen Ende der Hülle offen enden. Die Funktionskanäle sind so angeordnet, dass diese zwischen der Außenseite des Endoskops und der Innenseite der Hülle positioniert sind.

Durch die Integration der Funktionskanäle, die beispielsweise zur Biopsieprobenentnahme dienen können und 1 oder für das Spülmittel für die optischen Elemente verwendet werden können, in der Schutzhülle, wird die Verunreinigung des Endoskopschafts und der darin liegenden Kanäle verhindert. Bei diesem Hygieneschutz werden die an dem Hygieneschutz vorgesehenen FunktionsKanäle statt der in dem Endoskop vorgesehenen Kanäle verwendet. Ein Nachteil, den diese Art des Hygieneschutzes mit sich bringt, besteht darin, dass das Endoskop im Einsatz gekrümmt werden muss, um dem Verlauf der. Körperhöhlen folgen zu können, diese observieren und behandeln zu können. Da die Funktionskanäle an der Außenseite des Endoskopschaftes geführt werden, werden diese bei einer Krümmung des Endoskops gestaucht oder gestreckt. Im letzteren Fall kann es sogar zum Abreißen der Kanäle von dem distalen Ende der Hülle und somit zum Eintritt von kontaminierten Körperflüssigkeiten in die Hülle und zur Verunreinigung des Endoskops kommen. Zudem können die Funktionskanäle bei der Führung entlang des Endoskopschaftes bei dem Durchführen durch Körperhöhlen geringen Durchmessers beschädigt oder verschlossen werden. Weiterhin bewirken Kanäle, die zwischen dem Endoskop und der Schutzhülle eingebracht sind, eine erhöhte Reibung beim Aufziehen des Hygieneschutzes sowie beim Entfernen beziehungsweise Abziehen des Hygieneschutzes von dem Endoskop. Schließlich bilden sich aufgrund der außen angeordneten Funktionskanäle Falten an der Schutzhülle. Durch diese Faltenbildung wird die Handhabung und Hygiene des Hygieneschutzes beeinträchtigt.

In der US 5,419,311 A ist ein weiterer Überzug für ein Endoskop beschrieben. Auch bei diesem Überzug sind die Funktionskanäle so angeordnet, dass diese nach dem Aufziehen des Überzuges auf den Schaft des Endoskops neben diesem liegen.

In der US 4,741,326 A ist eine Hülle für einen Endoskopschaft beschrieben, bei dem an einer Endplatte ein Schlauch zum Einbringen in einen Arbeitskanal vorgesehen ist. Weiterhin kann an der Endplatte der Hülle eine Düse vorgesehen sein, über die Wasser und Luft zu einem optischen Bereich der Endplatte geleitet werden können. Die Düse kann mit einem weiteren Schlauch verbunden werden, der in der Hülle neben dem Endoskopschaft geführt wird. Alternativ kann die Düse so angeordnet sein, dass diese mit dem Wasser-Luftkanal des Endoskopschaftes überlagert und so Wasser und Luft zu dem optischen Bereich geführt werden kann.

Der Erfindung liegt die Aufgabe zugrunde, eine Möglichkeit zu schaffen, mit der auf einfache und zuverlässige Weise die Verunreinigung eines Endoskops während der Endoskopie vermieden werden kann. Die Erfindung ist durch die Merkmale des Anspruchs 1 definiert.

Gemäß einem ersten Aspekt wird die Aufgabe durch einen Hygieneschutz für ein Endoskop mit mindestens einem Arbeitskanal gelöst, wobei der Hygieneschutz an seinem distalen Ende eine Kappe mit einem daran vorgesehenen Arbeitskanalschlauch und eine sich an die Kappe anschließende Schutzhülle umfasst, wobei der Arbeitskanalschlauch an der Kappe zu dem Umfang der Kappe beabstandet vorgesehen ist. Der Hygieneschutz zeichnet sich dadurch aus, dass in der Kappe ein Sprühkopf angeordnet ist, sich in axialer Richtung des Hygieneschutzes zumindest proximal über die Kappe hinaus erstreckt und der Sprühkopf an dessen proximalen Ende ein Ventil aufweist.

Als Kappe wird im Sinne dieser Erfindung das stirnseitige Teil des Hygieneschutzes bezeichnet. Dieses ist vorzugsweise durch einen Hohlzylinder gebildet, der an dem distalen Ende verschlossen ist. Soweit nicht anders angegeben wird bei Bezugnahme auf die Kappe deren Stirnseite bezeichnet. Es liegt auch im Rahmen der Erfindung die Kappe als flache Scheibe auszugestalten, die die Stirnseite des Hygieneschutzes darstellt.

Als Arbeitskanal wird im Sinne dieser Erfindung der Kanal des Endoskops bezeichnet, über den Instrumente beispielsweise zur Entnahme von Gewebeproben (Biopsie) oder zur Durchführung kleinerer Operationen eingeführt werden können. Weiterhin dient der Arbeitskanal auch zum Absaugen von Körperflüssigkeiten. Erfindungsgemäß ersetzt der Arbeitskanalschlauch den Arbeitskanal für diese Zwecke. Der Arbeitskanal liegt innerhalb des Endoskops, das heißt innerhalb des Endoskopschaftes.

Der an dem Hygieneschutz vorgesehene Arbeitskanalschlauch kann an einem Durchlass an der Kappe, der dem Inneridurchmesser des Arbeitskanalschlauches entspricht, angebracht sein oder sich durch einen Durchlass in der Kappe, der dem Außendurchmesser des Arbeitskanalschlauches entspricht, hindurch bis zu der distalen Oberfläche der Kappe erstrecken und gegebenenfalls geringfügig über diese Oberfläche überstehen. Der Arbeitskanalschlauch kann an der Kappe befestigt sein, oder mit dieser integral hergestellt sein.

Indem der Arbeitskanalschlauch in einem Abstand zu dem Umfang der Kappe des Hygieneschutzes vorgesehen ist, liegt dieser in einem geringen Abstand zu dem Mittelpunkt der Kappe. Durch den Mittelpunkt verläuft die Faser des Endoskops, auf die bei einer Krümmung des Endoskops die geringsten Kräfte wirken und die im Folgenden auch als neutrale Faser bezeichnet wird. Je kleiner der Abstand zu der neutralen Faser ist, umso weniger wird der Arbeitskanalschlauch bei einer Krümmung des Endoskops gestreckt beziehungsweise gestaucht. Ein Abreißen des Arbeitskanalschlauches von der Kappe kann somit verhindert werden.

Der Arbeitskanalschlauch kann an der Kappe in der Position vorgesehen sein, die der Position des Arbeitskanals eines in die Schutzhülle des Hygieneschutzes eingeführten Endoskops entspricht. Auf diese Weise kann der Arbeitskanal des Endoskops als Führung für den Arbeitskanalschlauch dienen und ein gesondertes Einbringen einer Nut oder einer anderen Führung für den Arbeitskanalschlauch in das Endoskop kann entfallen. Hierdurch kann der Hygieneschutz für herkömmliche Endoskope mit Arbeitskanal verwendet werden, ohne dass eine Modifikation an dem Endoskop erforderlich ist. Weiterhin ist die Führung des Arbeitskanalschlauches in dem Arbeitskanal des Endoskops von Vorteil, da der Arbeitskanalschlauch durch das Endoskop gegen von außen einwirkende Kräfte, insbesondere radial gerichtete Druckkräfte, die beim Durchführen des Endoskops durch eine Körperhöhle geringen Durchmessers auftreten können, geschützt ist. Aus diesem Grund kann das Material des Arbeitskanalschlauches eine geringere Wandstärke und Festigkeit aufweisen, als dies bei einem außen geführten Schlauch der Fall wäre. Da der Hygieneschutz nach jeder Verwendung entsorgt werden muss, können durch diese geringere Materialstärke der Schlauchwand des Arbeitskanalschlauches die Materialkosten und der Entsorgungsbedarf minimiert werden.

Weiterhin wird durch die Anordnung des Arbeitskanalschlauches an der Position des Arbeitskanals eine Verdrehsicherung zwischen dem Hygieneschutz und dem Endoskop geschaffen. Die Ausrichtung des Hygieneschutzes, insbesondere der Kappe zu der distalen Stirnseite des Endoskops ist von besonderer Bedeutung, da in der Regel nur ein Teil der Kappe für den Durchlass optischer Wellen, insbesondere Lichtwellen ausgelegt ist. Verdreht sich eine solche in der Regel nicht rotationssymmetrisch aufgebaute Kappe gegenüber dem Endoskop kann es zu einem zumindest teilweise Verdecken der optischen Elemente des Endoskops kommen und die Untersuchung oder Operation behindern.

Schließlich wird durch die Anordnung des Arbeitskanalschlauches in einer Position, die der Position des Arbeitskanals entspricht, das Aufbringen des Hygieneschutzes erleichtert. Während bei bekannten Hüllen nach dem Stand der Technik der behandelnde Arzt bei dem Aufbringen der Hülle die Ausrichtung der Kappe mit den optischen Elementen des Endoskops überwachen musste, kann bei dem erfindungsgemäßen Hygieneschutz dieser Ausrichtungsschritt entfallen.

Erfindungsgemäß ist in der Kappe ein Sprühkopf angeordnet beziehungsweise in diese integriert. Der Sprühkopf erstreckt sich in axialer Richtung des Hygieneschutzes zumindest proximal über die Kappe hinaus. Auch distal kann der Sprühkopf über die Kappe hervorstehen. Es ist aber auch möglich, dass der Sprühkopf in der Kappe endet und somit in distaler Richtung nicht über die Kappe hervorsteht.

Als proximale Richtung wird die Richtung von dem distalen zu dem proximalen Ende des Hygieneschutzes und als distale Richtung, die Richtung von dem proximalen zu dem distalen Ende des Hygieneschutzes bezeichnet.

Steht der Sprühkopf in distaler Richtung über die Kappe hinaus, so kann in diesem Bereich eine auf die Stirnseite der Kappe gerichteten Öffnung vorgesehen sein, über die Spülmittel, wie beispielsweise Wasser oder Luft auf den Bereich der optischen Elemente geleitet werden kann. Endet der Sprühkopf in der Kappe, so ist dieser unmittelbar angrenzend an den Bereich der optischen Elemente angeordnet, so dass auch bei dieser Ausführungsform das Spülmittel auf diesen Bereich aufgebracht werden kann.

Der Überstand in proximaler Richtung hingegen dient zur Verbindung mit einer Zuführung für Spülmittel. Insbesondere bei einer bevorzugten Anordnung des Sprühkopfes, in der dieser beabstandet zu dem Umfang der Kappe ist, kann der proximale Überstand mit dem Spülkanal des Endoskops ausgerichtet zein. In diesem Fall dient der Überstand in proximaler Richtung dem Eintritt des Sprühkopfes in den Spülkanal des Endoskops. Durch den Eintritt in den Spülkanal wird eine weitere Verdrehsicherung zwischen dem Hygieneschutz und dem Endoskop geschaffen. Zudem dichtet der Überstand den Übergang von dem Spülkanal zu dem Sprühkopf ab und verhindert so ein Eintreten von Spülmittel in den Zwischenraum zwischen dem distalen Ende des Endoskops und der Kappe des Hygieneschutzes.

Gemäß einer bevorzugten Ausführungsform reicht der Sprühkopf mit seinem proximalen Ende in das distale Ende eines Spülkanals eines in den Hygieneschutz eingeführten Endoskops und endet in dem Sprühkanal. Dies bedeutet, dass die Länge des proximalen Überstandes des Sprühkopfes wesentlich geringer ist als die Länge der Schutzhülle des Hygieneschutzes. Für den Sprühkanal ist eine Überbrückung des zwischen dem distalen Ende des Endoskops und der Innenseite der Kappe durch den Sprühkopf ausreichend. Indem der Sprühkopf so kurz ausgestaltet ist, dass dieser in dem Spülkanal endet, wird das Aufbringen des Hygieneschutzes vereinfacht. Es ist insbesondere nicht erforderlich einen zweiten Schlauch in einen Kanal des Endoskops zu schieben. Zudem sind die Materialkosten bei einem kurzen Sprühkopf geringer, was, wie oben beschrieben, für den erfindungsgemäßen Hygieneschutz, der ein Einmalprodukt darstellt, von besonderem Vorteil ist.

Erfindungsgemäß ist an dem Sprühkopf an dessen proximalen Ende ein Ventil vorgesehen. Dieses Ventil, das als Sperrventil mit einer Sperrrichtung ausgestaltet ist, kann beispielsweise ein Entenschnabel- oder ein Domventil darstellen. Durch ein solches Ventil kann zwar die Zuführung von Spülmittel zu dem Sprühkopf gewährleistet, ein Eintreten von kontaminierten Körperflüssigkeiten oder anderer verkeimter Flüssigkeiten aber verhindert werden. Der Spülkanal muss daher nach der Verwendung des Endoskops nicht gereinigt werden.

Die Schutzhülle des Hygieneschutzes kann unterschiedliche Formen aufweisen. Somit kann die Schutzhülle beispielsweise einen Überzug aus Silikon, Latex oder einem anderen flüssigkeitsdichten Material darstellen, wobei dieses Material vor der Aufbringung auf das Endoskop aufgerollt vorliegen kann. In diesem Fall wird der Arbeitskanalschlauch in den Arbeitskanal eingebracht und der Überzug, der an der Kappe befestigt ist, befindet sich, wie ein Kondom, im aufgerollten Zustand. Zum Aufbringen des Überzuges auf das Endoskop wird dann der Überzug über das Endoskop mit dem darin angeordneten Arbeitskanalschlauch abgerollt, so dass das Endoskop vollständig bedeckt ist. Um das Endoskop wieder zu entfernen, wird der Überzug wieder aufgerollt.

Eine weitere Alternative für die Ausgestaltung der Schutzhülle ist beispielsweise ein Überzug, der mit Vakuumtechnik aufgebracht wird. In diesem Fall kann das Material des Überzuges beispielsweise Polyethylen, Polypropylen, Polyurethan, Silikon oder Latex sein.

Die Schutzhülle wird an der Kappe des Hygieneschutze befestigt und liegt nach dem Einführen des Endoskops lose an dem Endoskopschaft an. Anschließend wird der Überzug etwas mit der Hand angezogen, so dass das ganze Endoskop bedeckt ist. Mit einer Spritze und/oder Pumpe wird ein Vakuum erzeugt, das den Überzug eng und fest an die Endoskopoberfläche zieht. Um den Überzug wieder zu entfernen wird Luft mit einer Spritze und/oder Pumpe eingebracht. Somit löst sich der Überzug wieder von dem Endoskop und dieses kann entfernt werden.

Eine weitere Möglichkeit der Ausgestaltung der Schutzhülle besteht in der Verwendung eines so genannten Memory-Überzuges, das heißt eines Überzuges mit Formerinnerungsvermögen. Vorzugsweise wird ein Überzug mit temperaturabhängigem Formerinnerungsvermögen eingesetzt. Als Material kann beispielsweise Kunststoff verwendet werden, der auf Wärme und Kälte reagiert. Bei Erwärmung wird dieser sich dehnen, bei Kälteeinwirkung wird er sich zusammenziehen.

In diesem Fall wird der Überzug beim Einbringen des Endoskops mit der Hand gestreckt, so dass das gesamte Endoskop bedeckt ist. Durch Kälteeinwirkung, zum Beispiel kaltes Wasser, zieht sich der Überzug zusammen. Um das Endoskop wieder zu entfernen kann durch Wärmeeinwirkung, zum Beispiel warmes Wasser, der Überzug gedehnt werden.

Weiterhin kann ein Wickelüberzug als Schutzhülle verwendet werden. In diesem Fall besteht die Schutzhülle aus einem Tape oder einer Binde. Diese kann aus Kunststoffen wie beispielsweise Polyurethan, Polypropylen oder Polytetrafluoroethylen (PTFE) bestehen.

In diesem Fall wird die Schutzhülle nach dem Einbringen des Arbeitskanalschlauches in das Endoskop bis zum proximalen Ende des Endoskopschaftes gewickelt, so dass der gesamte Endoskopschaft bedeckt ist. Zum Entfernen wird der Wickelüberzug wieder abgewickelt. Der Wickelüberzug kann zum besseren Halt auf dessen Innenseite mit einem Klebstoff beschichtet sein.

Vorzugsweise weist die Schutzhülle mindestens zwei Lagen auf. Die Lagen weisen jeweils eine Schlauchform auf und sind ineinander angeordnet. Mit dem zweilagigen Aufbau wird es möglich die Materialeigenschaften zweier unterschiedlicher Materialien zu kombinieren. Insbesondere kann durch die innere Lage die Form der Schutzhülle bestimmt und durch die äußere Lage die Abdichtung gegen Kontakt des Endoskops mit kontaminierten Flüssigkeiten verhindert werden.

Zumindest eine Lage der Schutzhülle kann so ausgestaltet sein, dass diese bei axialer Verlängerung eine Verringerung des Durchmessers der Schutzhülle bewirkt. Eine solche Lage kann insbesondere durch ein Gitter realisiert werden. Da die Schutzhülle aus zwei Lagen besteht, kann ein solches Gitter, das alleine nicht den gewünschten Schutz liefern würde, verwendet werden und die weitere Lage undurchlässig gegenüber Luft- und Flüssigkeitseintritt ausgestaltet sein. Die zweite, äußere Lage kann beispielsweise aus Latex oder Silikon bestehen.

Die Lage, die die Querschnittsveränderung bei Längenänderung ausführt, kann elastisch ausgestaltet sein. Dies bedeutet, dass die Querschnittsverminderung bei Verringerung der Länge der Lage wieder rückgängig gemacht wird. Dies wird auch als elastisch selbstexpandierender Effekt bezeichnet. Die Verwendung einer solchen elastischen Lage weist den Vorteil auf, dass die Schutzhülle zum einen einfach auf das Endoskop aufgebracht und auch leicht wieder von dieser entfernt werden kann. Besonders bevorzugt ist der Durchmesser der Lage im entspannten Zustand größer als im angespannten Zustand und ist im entspannten Zustand größer als der Außendurchmesser des Endoskops. Auf diese Weise kann das distale Ende des Endoskops in die Schutzhülle eingebracht und bis zu der Kappe des Hygieneschutzes vorgeschoben werden. Hierbei sind keine Reibungskräfte zwischen der Schutzhülle, insbesondere der inneren Lage, und dem Endoskop zu überwinden.

Ist der Teil des Endoskops, das heißt insbesondere zumindest der Teil des Endoskopschaftes, der in die Körperhöhle eingeführt werden soll, in der Schutzhülle aufgenommen, so kann durch Zug eine Verlängerung der Lage erzielt werden und auf diese Weise der Durchmesser der Schutzhülle verkleinert werden, bis diese an dem Endoskop anliegt. An der Schutzhülle kann eine Klemmvorrichtung zum Fixieren der Schutzhülle in dieser Position vorgesehen sein. Indem die Klemmvorrichtung an der Schutzhülle vorgesehen ist, kann jedes herkömmliche Endoskop mit diesem Hygieneschutz überzogen werden. Die Klemmvorrichtung, durch die eine radiale Kraft auf die auf dem Endoskop aufgebrachte Schutzhülle an deren proximalen Ende aufgebracht wird, kann beispielsweise ein Klettband sein, das um die Schutzhülle gewickelt und so befestigt werden. Wird die Klemmvorrichtung nach dem Gebrauch gelöst, verringert sich aufgrund der Elastizität der Schutzhülle deren Länge und dadurch wird der Durchmesser der Schutzhülle wieder die ursprüngliche Größe annehmen. Das Endoskop kann in diesem Zustand leicht aus der Schutzhülle herausgenommen werden, ohne, dass das Endoskop mit der Außenseite der Schutzhülle in Kontakt kommt.

Die mindestens zwei Lagen können nur an der Kappe und an dem proximalen Ende der Schutzhülle miteinander verbunden sein, insbesondere aneinander befestigt sein. Hierdurch wird zum einen die Herstellung des Hygieneschutzes erheblich vereinfacht und dadurch die Herstellungskosten weiter gesenkt. Auch Material, insbesondere Klebstoff kann bei dieser Ausgestaltung eingespart werden. Insbesondere bei der Verwendung eines Gitters als eine der Lagen könnte es bei einer Befestigung, insbesondere einem Aufkleben der weiteren Lage über die gesamte Länge dazu kommen, dass bei Vergrößerung der Abstände der Gitterstäbe die daran befestigte Lage zu sehr beansprucht wird und reißt. Wird als weitere Lage beispielsweise eine elastische Folie gewählt und diese nur an dem proximalen und distalen Ende mit dem Gitter verbunden, so kann die elastische Folie über die gesamte Länge an dem Gitter anliegen, und durch diese große Anzahl an Kontaktstellen mit dem Gitter ein Verrutschen der Folie auf dem Gitter verhindert werden. Zu diesem Zweck wird der Durchmesser der Lage im entspannten Zustand kleiner als der Durchmesser des Gitters im entspannten Zustand gewählt.

Die Kappe des erfindungsgemäßen Hygieneschutzes weist vorzugsweise einen Sichtbereich auf, der durch eine Folie abgedeckt ist. Als Sichtbereich wird der Bereich bezeichnet, der bei der Benutzung des Endoskops über den optischen Elementen, beispielsweise lichtleitende und lichtgebende Elementen, angeordnet ist. Dieser Bereich muss aus einem für optische Wellen durchlässigen Material bestehen. Der Sichtbereich kann daher durch eine Glasscheibe, eine Kunststoffplatte oder der Gleichen gebildet sein. Es hat sich jedoch gezeigt, dass bei der Verwendung von Materialien für den Sichtbereich, die eine gewisse Dicke aufweisen, Reflektionen an dem Sichtbereich auftreten, die ein Erkennen des zu betrachtenden Bereiches der Körperhöhle erschwert oder ganz unmöglich macht. Erfindungsgemäß kann bei der Verwendung einer Glasscheibe oder Kunststoffplatte zur Überbrückung des Zwischenraumes eine Flüssigkeit eingebracht werden.

Insbesondere wird eine Folie in dem Sichtbereich verwendet. Als Folie wird in diesem Zusammenhang ein Material bezeichnet, das eine sehr geringe Materialstärke, beispielsweise im Bereich von 0,01mm bis 0,5mm aufweist. Es hat sich gezeigt, dass bei der Verwendung einer Folie eine Reflektion des Lichtes nicht auftritt und damit auf das Einbringen einer optischen Flüssigkeit verzichtet werden kann. Als Material für die Folie kann beispielsweise Polyethylen, Polypropylen, Polyurethan oder Plexiglas verwendet werden.

An dem proximalen Ende des Arbeitskanalschlauches kann erfindungsgemäß ein Ventil, vorzugsweise ein Ventil mit einem Seitenzugang, befestigt sein. Dieses wird nach dem Einbringen des Arbeitskanalschlauches in den Arbeitskanal aufgebracht. Durch ein solches Ventil wird es möglich über den Arbeitskanal sowohl Instrumente, beispielsweise für die Entnahme von Biopsieproben einzubringen, als auch Flüssigkeiten von dem Bereich vor der Kappe abzusaugen. In diesen beiden Fällen muss der Arbeitskanal mit dem entsprechenden Zugang oder einer Absaugvorrichtung verbunden werden. Dies wird über das vorzugsweise vorgesehene Ventil mit Seitenzugang, der auch als Sideport des Ventils bezeichnet wird, realisiert. Hierbei ist der Seitenzugang des Ventils mit einer Absaugvorrichtung verbunden. Der Hauptkanal des Ventils, der an dessen proximalen Ende beispielsweise durch eine Membran abgedichtet ist, dient der Zuführung von medizinischen Instrumenten. In der Leitung zwischen dem Seitenzugang des Ventils und der Absaugvorrichtung, die im Folgenden auch als Absaugleitung bezeichnet wird, ist ein Absaugventil vorgesehen, über das die Absaugung aus dem Bereich vor der Kappe des Hygieneschutzes gesteuert werden kann.

Hierdurch werden der Arbeitskanalschlauch und die Absaugleitung ausschließlich aus Komponenten des Hygieneschutzes gebildet. Der in dem Endoskop vorgesehene Absaugkanal wird daher nicht genutzt und eine Reinigung dieses Absaugkanals kann dadurch entfallen.

Gemäß einer Ausführungsform ist an der Schutzhülle an deren proximalen Ende eine konische Verlängerung angebracht. Durch diese konische Verlängerung, die aus einem Kunststoff bestehen kann, beispielsweise aus Polypropylen oder Polyamid, kann auch der Bereich des Endoskops zwischen dem Seitenzugang beziehungsweise Sideport des Endoskops und dem Griff des Endoskops abgedeckt und vor Verunreinigung geschützt werden. An die konische Verlängerung kann sich eine Abdeckung anschließen, die über den Griff des Endoskops gezogen werden kann und dadurch auch eine Verunreinigung des Endoskopgriffes, des Manipulationsrades und des Absaug- und Spülventils verhindern kann.

Vorzugsweise ist an der konischen Verlängerung der Schutzhülle das Ventil zum Anschluss an das proximale Ende des Arbeitskanalschlauches vorgesehen. Nach dem Aufbringen der konischen Verlängerung auf dem Endoskop kann das Ventil mit dem durch den Arbeitskanal geführten Arbeitskanalschlauch verbunden und daran befestigt werden.

Gemäß einem weiteren Aspekt betrifft die Erfindung ein Endoskop mit Hygieneschutz, das sich dadurch auszeichnet, dass dieses einen erfindungsgemäßen Hygieneschutz aufweist. Bevorzugt ist das Endoskop so ausgestaltet, dass der Hygieneschutz eine Kappe aufweist, die in unmittelbarer Nähe des distalen Endes des Endoskops liegt, ein Arbeitskanalschlauch durch den Arbeitskanal des Endoskops geführt ist und die Länge des Arbeitskanalschlauchs größer als die Länge des Arbeitskanals ist. Der Arbeitskanal erstreckt sich von dem distalen Ende des Endoskopschaftes bis zu dem Seitenzuggäng beziehungsweise Sideport des Endoskops.

Neben den bereits beschriebenen Vorteilen des in dem Arbeitskanal geführten Arbeitskanalschlauchs, weist das erfindungsgemäße Endoskop den Vorteil auf, dass durch die große Länge des Arbeitskanalschlauches dieser aus dem Endoskop an der proximalen Seite herausgeführt werden kann. Hierdurch kann zum einen die Verbindung zwischen der Zuführungsstelle von medizinischen Instrumenten und der Entnahmestelle vor der Kappe des Hygieneschutzes vollständig durch einen Teil des Hygieneschutzes, insbesondere des Arbeitskanalschlauches, gebildet werden. Zudem kann über das Ventil eine externe Anbindung des Arbeitskanalschlauches an eine Absaugvorrichtung ermöglicht werden.

Vorzugsweise ist an dem proximalen Ende des Arbeitskanals ein Ventil mit einem Seitenzugang befestigt und der Seitenzugang mit dem Absaugventil des Endoskops verbunden ist. Der Seitenzugang des Ventils wird als Sideport des Ventils bezeichnet.

Gemäß einer Ausführungsform liegt zwischen dem distalen Ende des Endoskops und der Kappe des Hygieneschutzes bei der Benutzung des Endoskops Luft vor. Dies kann bei dem erfindungsgemäßen Endoskop insbesondere dadurch ermöglicht werden, dass als Material für das Sichtfenster eine Folie verwendet wird. In der bevorzugten Form liegt die Folie auf den optischen Elementen im direkten Kontakt auf. Die Folie kann beispielsweise von innen auf die Stirnseite der Kappe über eine Aussparung aufgeklebt sein. Hierdurch kann eine Reflektion an dem Sichtfenster vermieden werden ohne eine optische Flüssigkeit einbringen zu müssen.

Der Hygieneschutzes kann an einem Endoskop angebracht werden, indem das proximale Ende eines Arbeitskanalschlauchs des Hygieneschutzes in das distale Ende des Arbeitskanals eingeführt wird, durch den Arbeitskanal geschoben wird, bis das proximale Ende des Arbeitskanalschlauches über das proximale Ende des Arbeitskanals hervorsteht. Bei Einführen des Endoskops in den Hygieneschutz ist dieser noch nicht verunreinigt. Insofern kann der Arzt nach dem Einfädeln des Arbeitskanalschlauches in den Arbeitskanal den Hygieneschutz mit einer Hand an der Kappe halten und das Endoskop weiter schieben.

Nach dem Einführen des Endoskopschafts in die Schutzhülle, wird diese in axialer Richtung gestreckt, bis deren Durchmesser dem Außendurchmesser des Endoskopschafts entspricht und in dieser Lage an dem proximalen Ende der Schutzhülle an dem Endoskopschaft fixiert.

Das proximale Ende des Arbeitskanälschlauches wird vorzugsweise in ein Ventil eingeführt, über das eine Verbindung einer Absaugvorrichtung und einer Zuleitung für medizinische Instrumente vorliegt.

Vorteile und Merkmale, die bezüglich des Hygieneschutzes beschrieben werden, gelten - soweit anwendbar - entsprechend für das erfindungsgemäße Endoskop, sowie jeweils umgekehrt.

Die Erfindung wird im Folgenden erneut unter Bezugnahme auf die beiliegenden Zeichnungen genauer beschrieben. Es zeigen:
Figur 1: eine schematische perspektivische Ansicht einer Ausführungsform eines erfindungsgemäßen Endoskops mit einem erfindungsgemäßen Hygieneschutz;
Figur 2: eine schematische Schnittansicht einer Ausführungsform eines erfindungemäßen Endoskops mit einem erfindungsgemäßen Hygieneschutz;
Figur 3: eine schematische Schnittansicht durch den distalen Bereich einer Ausführungsform eines erfindungsgemäßen Hygieneschutzes;
Figur 4: eine schematische Schnittansicht durch den distalen Bereich einer Ausführungsform eines erfindungsgemäßen Endoskops mit einem erfindungsgemäßen Hygieneschutz;
Figur 5: eine schematischen Frontansicht einer Ausführungsform eines erfindungsgemäßen Endoskops mit einem erfindungsgemäßen Hygieneschutz;
Figur 6: eine schematische Schnittansicht einer Kappe eines erfindungsgemäßen Hygieneschutzes;
Figur 7: eine schematische Schnittansicht einer Ausführungsform des erfindungsgemäßen Absaugventils; und
Figur 8: eine schematische Ansicht einer weiteren Ausführungsform des erfindungsgemäßen Hygieneschutzes.

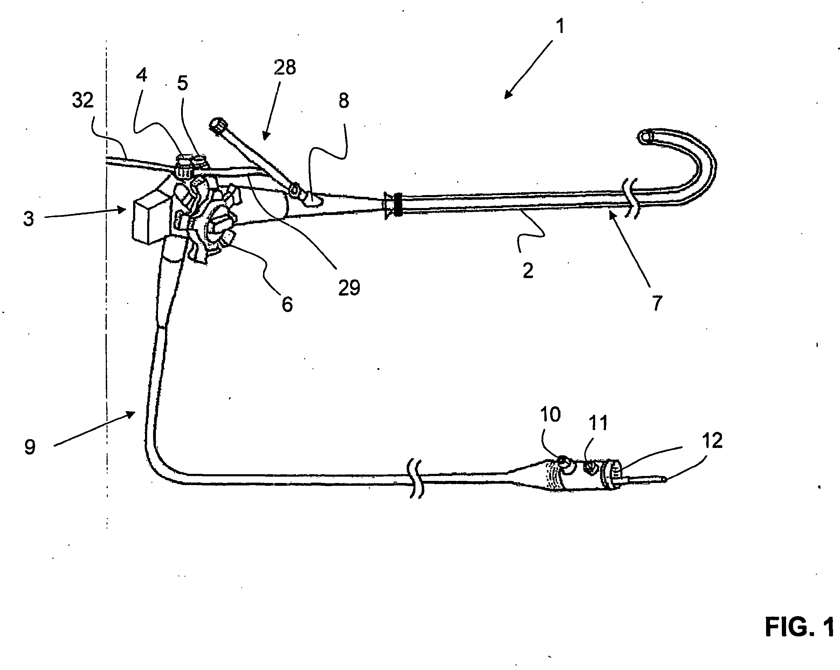

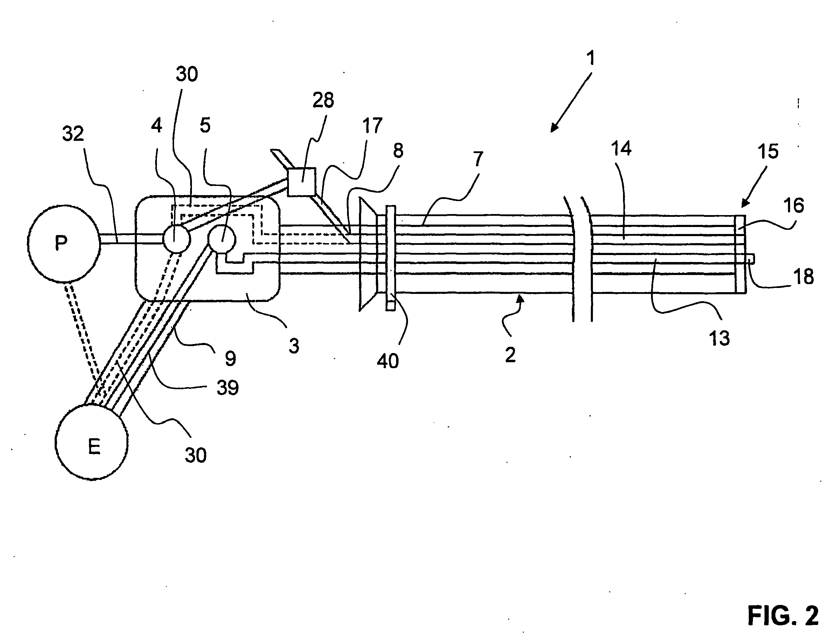

In Figur 1 ist eine Ausführungsform eines erfindungsgemäßen Endoskops 1 mit einem erfindungsgemäßen Hygieneschutz 2 gezeigt. Das Endoskop 1 besitzt einen Griff 3, an dem ein Absaugventil 4 und ein Spülventil 5 vorgesehen sind. Von den Ventilen 4 und 5 ist in der gezeigten Ansicht jeweils nur der oben auf dem Griff 3 angeordnete Betätigungsknopf zu erkennen. An der Seite besitzt der Griff 3 ein Manipulationsrad 6 mittels dessen der Schaft 7 des Endoskops 1 bewegt werden kann. Auf dem Endoskopschaft 7 ist der erfindungsgemäße Hygieneschutz 2 aufgebracht. Am distalen Ende des Endoskopschaftes 7 ist ein Zugang 8, der auch als Sideport des Endoskops 1 bezeichnet wird, zum Einführen von medizinischen Instrumenten (nicht dargestellt) vorgesehen. An der Unterseite des Griffes 3 ist eine Anschlussleitung 9 zur Verbindung des Endoskops 1 mit der Einheit E zum Setreiben des Endoskops 1 (sieht Figur 2) vorgesehen. Durch die Anschlussleitung 9 verlaufen eine Wasser- und Luftleitung, eine Absaugleitung sowie Optikleitungen. Die Wasser- und Luftleitung tritt an dem Ende der Anschlussleitung 9, das mit der Einheit E verbunden wird, seitlich heraus und geht in einen Wasser-Luft-Anschluss 10 über. Ebenfalls ist an diesem Ende der Anschlussleitung 9 seitlich ein Absauganschluss 11 vorgesehen, der den Austritt der Absaugleitung in der Anschlussleitung 9 darstellt. An dem axialen Ende der Anschlussleitung 9 sind Anschlüsse 12 für die Optikelemente vorgesehen.

An dem Sideport 8 des Endoskops 1 ist ein Ventil 28 mit einem Seitenzugang beziehungsweise Sideport 29 vorgesehen, dessen Funktionsweise später genauer erläutert wird.

Der innere Aufbau des Endoskops 1 wird im Folgenden unter Bezugnahme auf Figur 2 beschrieben. Von dem Spülventil 5 im Griff 3 des Endoskops 1 erstreckt sich über die Länge des Endoskopschaftes 7 ein Spülkanal 13. Parallel zu dem Spülkanal 13 verläuft über die Länge des Endoskopschaftes 7 ein Arbeitskanal 14. Dieser Arbeitskanal 14 dient zum einen der Führung von Instrumenten zu dem distalen Ende des Endoskopschaftes 7 und zum anderen zum Absaugen von Sekret und Geweberesten. Der Arbeitskanal erstreckt sich von dem Sideport 8 des Endoskops 1 bis zum distalen Ende des Endoskopschaftes 7. Um diesen Arbeitskanal 14 auch zum Absaugen verwenden zu können, verläuft bei herkömmlichen Endoskopen durch das proximale Ende des Endoskopschaftes 7, den Griff 3 und die Anschlussleitung 9 ein Absaugkanal 30. Dieser Absaugkanal 30 ist in Figur 2 durch die gestrichelte Linie angedeutet und geht an dem Sideport 8 in den Arbeitskanal 14 über.

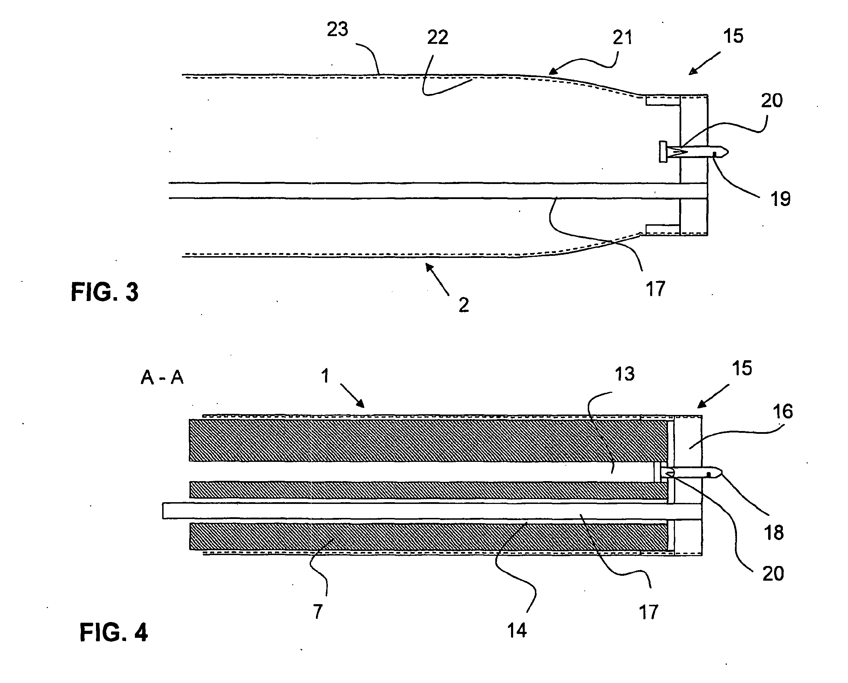

An dieses Endoskop 1 ist der erfindungsgemäße Hygieneschutz 2 einbeziehungsweise aufgebracht, dessen Aufbau sich insbesondere aus Figur 3 ergibt. Der Hygieneschutz 2 besteht aus einer Kappe 15, die im Wesentlichen eine Topfform aufweist. Die Stirnseite der Kappe 15 stellt im Wesentlichen eine Scheibe 16 dar. Die Scheibe 16 ist von einem Arbeitskanalschlauch 17 durchdrungen und mit diesem fest verbunden. Das distale offene Ende des Arbeitskanalschlauches 17 endet in der dargestellten Ausführungsform in dem distalen Ende der Scheibe 16. Weiterhin ist in der Scheibe 16 ein Sprühkopf 18 vorgesehen. Dieser ragt in der dargestellten Ausführungsform über die distale Seite und die proximale Seite der Scheibe 16 hinaus. Auf der distalen Seite ist in dem Sprühkopf 18 seitlich eine Sprühöffnung 19 vorgesehen und das distale Ende des Sprühkopfes 18 ist verschlossen. An dem proximalen Ende des Sprühkopfes 18 ist ein Sperrventil 20 vorgesehen, das in der Figur 3 ein Entenschnabelventil darstellt.

An der Kappe 15 ist eine Schutzhülle 21 an deren Umfang befestigt. Die Schutzhülle 21 besteht aus zwei Lagen 22 und 23, wobei die innere Lage 22 ein elastisches Gitter darstellt, das bei Vergrößerung der Länge einen reduzierten Durchmesser aufweist. Die äußere Lage 23 stellt in der dargestellten Ausführungsform eine Latexfolie beziehungsweise Latexschlauch dar. An der Kappe 15 besitzt die Schutzhülle 21 den Durchmesser der Kappe 15 und erweitert sich von dieser in axialer Richtung auf einen maximalen Durchmesser. Dieser Durchmesser ist größer als der Außendurchmesser eines in den Hygieneschutz 2 einzuführenden Endoskops 1, insbesondere des Endoskopschaftes 7. Wie in Figur 1 und 2 gezeigt, ist an dem proximalen Ende der Schutzhülle 21 eine Klemmvorrichtung, die in der dargestellten Ausführungsform ein Klettband 40 darstellt, vorgesehen. Mittels dieses Klettverschlusses kann eine auf die gewünschte Länge, in der der Durchmesser der Schutzhülle 21 dem Außendurchmesser des Endoskopschaftes 7 entspricht, das heißt an diesem anliegt, gezogene Schutzhülle 21 an dem Endoskop 1 fixiert werden. Es hat stich gezeigt, dass ein um die Schutzhülle 21 gewickeltes Klettband 40 eine ausreichende Kraft aufbringt, um die Schutzhülle 21 auch bei Verwendung des Endoskops 1 in der Position zu halten.

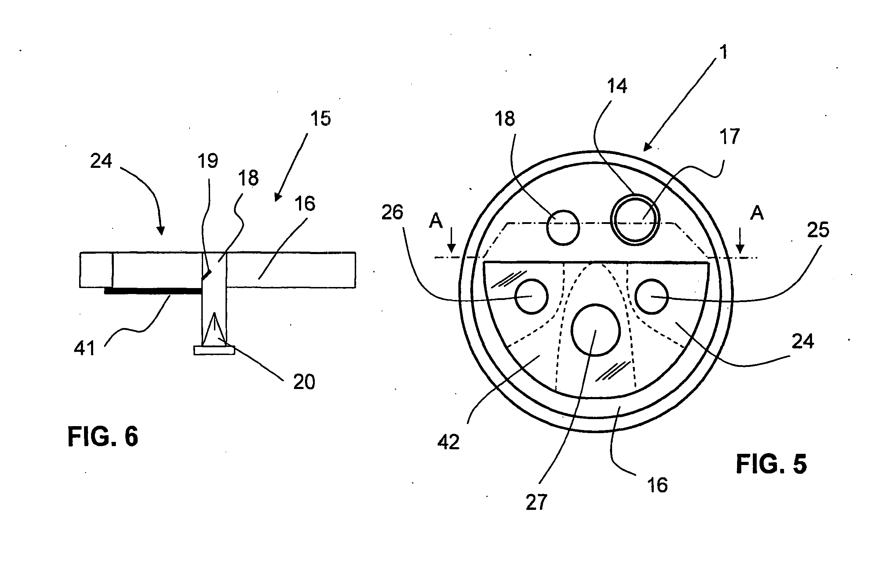

In Figur 4 ist eine Ausführungsform des Hygieneschutzes 2 in der auf dem Endoskopschaft 7 aufgebrachten Position gezeigt. Figur 4 zeigt einen Schnitt durch das Endoskop 1 entlang der Schnittlinie A - A in Figur 5. Der Arbeitskanalschlauch 17 ist in den Arbeitskanal 14 des Endoskops 1 eingebracht und der Sprühkopf 18 ragt in den Spülkanal 13 des Endoskops 1. Die Ausführungsform des Hygieneschutzes 2 entspricht im Wesentlichen der in Figur 3 gezeigten Ausführungsform, allerdings ist in der Figur 4 ein Domventil als Sperrventil 20 zum Absperren des Spülkanals 13 vorgesehen. Die Schutzhülle 21 liegt an der Außenseite des Endoskopschafts 7 an.

In Figur 5 ist die Frontansicht der Stirnseite des Endoskops 1 mit aufgebrachtem Hygieneschutz 2 gezeigt. In dieser Ansicht sind das offene distale Ende des Arbeitskanalschlauchs 17 in dem Arbeitskanal 14 und das geschlossene distale Ende des Sprühkopfes 18 zu erkennen. In der Scheibe 16 der Kappe 15 ist weiterhin ein Sichtfenster 24 vorgesehen. Dieses weist in der dargestellten Ausführungsform im Wesentlichen die Form eines Halbkreises auf. In dem Sichtfenster 24 ist eine Folie 41 angeordnet, die dieses abdeckt. Durch das Sichtfenster 24 lassen sich die optischen Elemente 25, 26 und 27 des Endoskops 1 erkennen. Dies sind insbesondere Leuchten 25 und 26, sowie ein Lichtleiter 27, über den Bilder von dem Bereich vor der Scheibe 16 an den Griff 3 und von dort an bildverarbeitende Einrichtungen (nicht dargestellt) übermittelt werden können. Die Folie 41 ist vorzugsweise an der Innenseite der Scheibe 16 befestigt. In dem Sichtfenster 24 können zusätzlich Stege 42 vorgesehen sein, die das Sichtfenster 24 in Bereichen Abdecken, an denen keines der optischen Elemente 25, 26 und 27 an dem distale Ende des Endoskops 1 liegen. Die Stege 42 sind in der Figur 5 durch gestrichelte Linien angedeutet und können einen Teil der Scheibe 16 darstellen, das heißt mit dieser integral aufgebaut sein. Durch diese Stege 42 erhält die Scheibe 16 eine zusätzliche Stabilität und die von einer Folie 41 abzudeckenden Bereiche werden verringert. Alternativ ist es auch möglich in der Scheibe 16 nur kleine Durchlässe einzubringen, die den Positionen der optischen Elemente 25, 26 und 27 entsprechen und einen größeren Durchmesser als diese Elemente 25, 26 und 27 aufweisen. Diese Durchlässe können dann mit der Folie 41 abgedeckt sein.

In der Figur 6 ist eine Ausführungsform der Scheibe 16 des Hygieneschutzes 2 gezeigt. Bei diesen Ausführungsform steht der Sprühkopf 18 nur proximale über die Scheibe 16 hervor. Distal endet der Sprühkopf 18 bündig mit der Oberfläche der Scheibe 16 oder ist sogar um einen gewissen Betrag gegenüber der Oberfläche proximal versetzt. Bei dieser Ausgestaltung ist der Sprühkopf 18 unmittelbar angrenzend an das Sichtfenster 24 angeordnet und die Sprühöffnung 19 ist auf dieses gerichtet. Die Anbringung der Folie 41 erfolgt auch bei dieser Ausgestaltung mittels Anbringung, beispielsweise Ankleben, an der Innenseite der Scheibe 16.

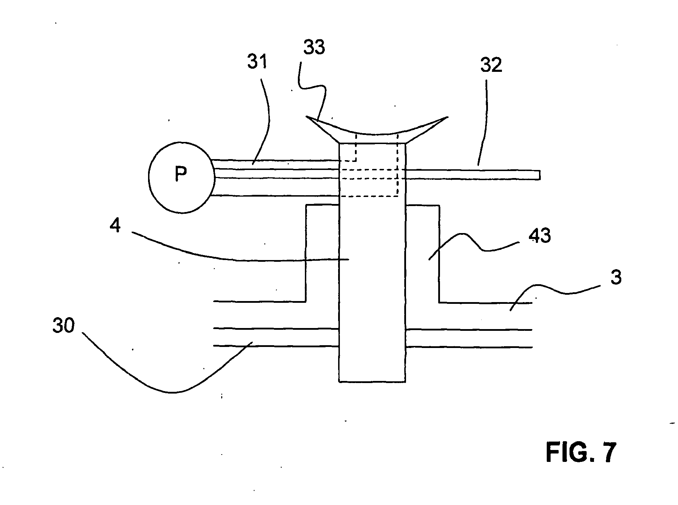

Die erfindungsgemäße Führung des Arbeitskanalschlauches 17 wird im Folgenden unter Bezugnahme auf die Figuren 1, 2 und 7 genauer erläutert. Beim Aufbringen des Hygieneschutzes 2 auf beziehungsweise in ein Endoskop 1 wird der Arbeitskanalschlauch 17 in den Arbeitskanal 14 in dem Endoskopschaft 7 von dem distalen Ende aus eingeführt. An dem Sideport 8 des Endoskops 1 gelangt der Arbeitskanalschlauch 17 in diesen Sideport 8 und verschließt damit den Absaugkanal 30, der in dem Griff 3 und der Anschlussleitung 9 verläuft. An dem proximalen Ende des Arbeitskanalschlauches 17 wird ein Ventil 28 aufgebracht. Ein Sideport 29 dieses Ventils 18 wird wiederum mit dem Absaugventil 4 des Griffes 3 verbunden. Eine bevorzugte Ausführungsform eines solchen Absaugventils 4 ist in der Figur 7 gezeigt. Das Absaugventil 4 ist in dem Ventilsitz 43, der an dem Griff 3 vorgesehen ist, aufgenommen. Der untere Bereich des Absaugventils 4 verschließt den Absaugkanal 30 in dem Griff 3. Es ist aber auch möglich, dass das Absaugventil 4 eine geringere Länge aufweist und so der Absaugkanal 30 offen bleibt. Dies ist wegen des in den Arbeitskanal 14 eingebrachten Arbeitskanalschlauch 17 unproblematisch, da dieser an, dem Sideport 8 den Absaugkanal 30 bereits verschließt.

Im oberen Bereich des Absaugventils 4, der über den Ventilsitz 43 für das Absaugventil 4 nach oben herausragt, ist das Absaugventil 4 von einer Absaugleitung 32 durchdrungen, die an einem Ende mit dem Sideport 29 des Ventils 28 und an dem anderen Ende mit einer Pumpe P verbunden ist. Weiterhin ist in dem Absaugventil 4 ein im wesentlicher L-förmiger Kanal 31 vorgesehen. Dieser reicht von dem Absaugventil 4 bis zu der Pumpe P. In dem Absaugventil 4 geht der Kanal 31 in einen vertikalen Teil über, der sich bis zur Oberseite des Absaugventils 4 erstreckt. An dieser Oberseite ist das Absaugventil 4 schalenartig ausgestaltet und bildet so eine Fingerauflage 33. Legt der behandelnde Arzt auf diese Auflage 33 seinen Finger, so wirkt der durch die Pumpe P erzeugte Sog ausschließlich auf die Absaugleitung 32. Ist der Kanal 31 hingegen nicht verschlossen, so wird der Sog auf die Absaugleitung 32 und auf den Kanal 31 wirken, wobei die wesentliche Saugleistung aufgrund des größeren Durchmessers über den Kanal 31 erfolgen und somit Umluft ansaugen wird.

Durch diesen Aufbau wird die Führung der Absaugleitung 32 vollständig außerhalb des Griffes 3 oder anderen Teilen des Endoskops 1 durchgeführt. Die Absaugleitung 32, das Absaugventil 4 und der Sideport 29 des Ventils 28 bilden einen Tei des Hygieneschutzes 2 und stellen somit einen Einwegartikel dar. Durch diese Ausgestaltung des Absaugventils 4 kann dieses in den bereits an dem Griff 3 vorgesehenen Ventilsitz 43 eingebracht werden und die Bedienung des Endoskops 1 wird, wie auch bei herkömmlichen Endoskopen, mit einer Hand ermöglicht. Zudem ist für den Arzt keine Umgewöhnung notwendig.

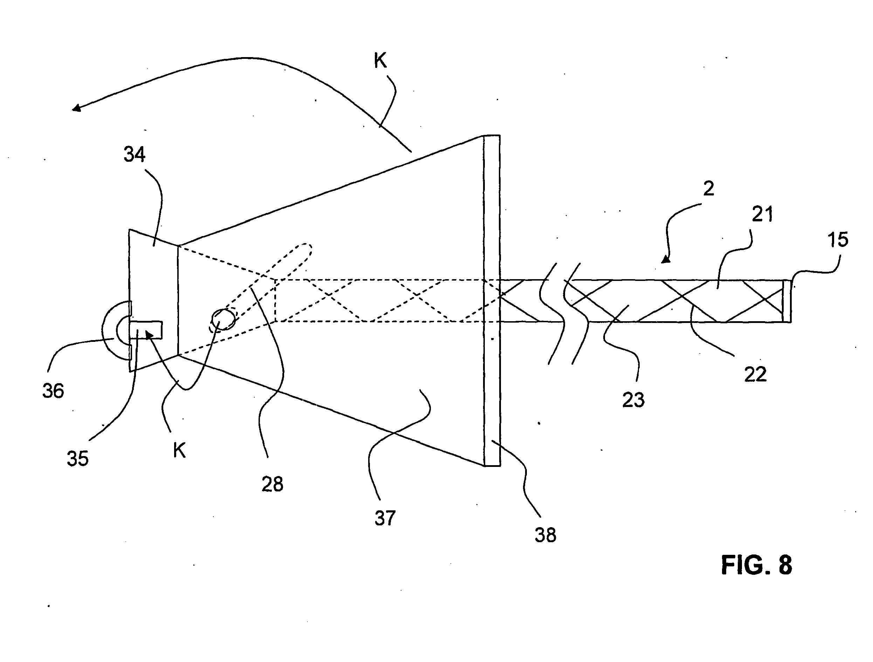

In der Figur 8 ist eine weitere Ausführungsform des Hygieneschutzes 2 dargestellt. Im distalen Bereich besitzt dieser den gleichen Aufbau, wie die bereits beschriebenen Hygieneschutze 2. Am proximalen Ende schließt sich bei der gezeigten Ausführungsform an die Schutzhülle 21 eine konische Verlängerung 34 an, die aus einer Kunststofffolie oder aus Latex bestehen kann. Durch die konische Form kann in dieser Verlängerung 34 das proximale Ende des Endoskopschaftes 7 aufgenommen werden. Am proximalen Ende der konischen Verlängerung 34 ist eine Aussparung 35 vorgesehen, durch die der Sideport 8 des Endoskops 1 geführt werden kann. Zur Fixierung der Schutzhülle 21 ist eine an zumindest einer Seite lösbar mit der konischen Verlängerung 34 verbundene Klemme 36, die ein Stoffband oder eine Metallklemme sein kann, vorgesehen. Distal ersetzt zu der Aussparung 35 ist eine Abdeckung 37 an der konischen Verlängerung 34 angebracht. Diese weist einen größeren Durchmesser als der größte Durchmesser der konischen Verlängerung 34 auf. An dem proximalen Ende der Abdeckung 37 ist ein Verschluss 38, der beispielsweise ein Zugband darstellen kann, vorgesehen. Weiterhin ist an der Abdeckung 37 das Ventil 28 befestigt.

Wird die Abdeckung 37 in der in der Figur 8 mit K bezeichneten Richtung umgeklappt, so kann diese den Griff 3 und einen Teil der Anschlussleitung 9 umgeben und an dieser mittels des Verschlusses 38 verschlossen werden. Auf diese Weise ist auch das Manipulationsrad 6 an dem Griff 3 vor Verunreinigungen geschützt. Dies ist von Bedeutung, da der behandelnde Arzt die medizinischen Instrumente, die bis über das distale Ende des Endoskops 1 geführt werden und mit Körperflüssigkeiten in Berührung kommen, mit der gleichen Hand betätigt, mit der er auch das Manipulationsrad 6 bedient. Durch die Abdeckung 37 kann somit auch eine Reinigung des Griffes 3 und des Manipulationsrades 6 entfallen.

Die Abmessung und das Material der Abdeckung 37 werden so gewählt, dass es für den Arzt weiterhin möglich ist das Manipulationsrad 6 zu bedienen.

Mit der vorliegenden Erfindung wird es somit möglich ein Endoskop 1 nach der Verwendung ohne einen Reinigungsschritt erneut verwenden zu können und dennoch eine Kontamination des nächsten Patienten zuverlässig verhindern zu können.

## Patentansprüche

1. Hygieneschutz für Endoskope mit mindestens einem Arbeitskanal (14), wobei der Hygieneschutz (2) an seinem distalen Ende eine Kappe (15) mit einem daran vorgesehenen Arbeitskanalschlauch (17) und eine sich an die Kappe (15) anschließende Schutzhülle (21) umfasst, wobei der Arbeitskanalschlauch (17) an der Kappe (15) zu dem Umfang der Kappe (15) beabstandet vorgesehen ist, wobei in der Kappe (15) ein Sprühkopf (18) angeordnet ist, der sich in axialer Richtung des Hygieneschutzes (2) über die Kappe (15) zumindest proximal hinaus erstreckt und **dadurch gekennzeichnet, dass** der Sprühkopf an dessen proximalen Ende ein Ventil (20) aufweist.

2. Hygieneschutz nach Anspruch 1, **dadurch gekennzeichnet, dass** der Sprühkopf (18) an der Kappe (15) zu dem Umfang der Kappe (15) beabstandet angeordnet ist.

3. Hygieneschutz nach Anspruch 1 oder 2, **dadurch gekennzeichnet, dass** der Sprühkopf (18) mit seinem proximalen Ende in das distale Ende eines Spülkanals (13) eines in die Schutzhülle (21) des Hygieneschutzes (2) eingeführten Endoskops (1) reicht und in dem Sprühkanal (13) endet.

4. Hygieneschutz nach einem der Ansprüche 1 bis 3, **dadurch gekennzeichnet, dass** an dem proximalen Ende des Arbeitskanalschlauches (17) ein Ventil (28), vorzugsweise ein Ventil mit einem Seitenzugang (29), befestigt ist.

5. Hygieneschutz nach Anspruch 4, **dadurch gekennzeichnet, dass** der Seitenzugang (29) mit einem Absaugventil (4) verbunden ist, das in einen Ventilsitz (43) eines in die Schutzhülle (21) des Hygieneschutzes (2) eingebrachten Endoskops (1), eingebracht werden kann.

6. Hygieneschutz nach einem der Ansprüche 1 bis 5, **dadurch gekennzeichnet, dass** an der Schutzhülle (21) eine konische Verlängerung (34) befestigt ist.

7. Hygieneschutz nach Anspruch 446, **dadurch gekennzeichnet, dass** an der konischen Verlängerung (34) der Schutzhülle (21) ein Ventil (28) zur Verbindung mit dem Arbeitskanalschlauch (17) vorgesehen ist.

8. Endoskop mit Hygieneschutz, **dadurch gekennzeichnet, dass** dieses einen Hygieneschutz (2) gemäß einem der Ansprüche 1 bis 7 aufweist.

9. Endoskop nach Anspruch 8, **dadurch gekennzeichnet, dass** der Hygieneschutz (2) eine Kappe (15) aufweist, die in unmittelbarer Nähe des distalen Endes des Endoskops (1) liegt, ein Arbeitskanalschlauch (17) durch den Arbeitskanal (14) des Endoskops geführt ist und die Länge des Arbeitskanalschlauchs (14) größer als die Länge des Arbeitskanals (14) ist.

10. Endoskop nach einem der Ansprüche 8 oder 9, **dadurch gekennzeichnet, dass** an dem proximalen Ende des Arbeitskanalschlauches (17) ein Ventil (28) mit einem Seitenzugang (29) befestigt ist und der Seitenzugang (29) mit dem Absaugventil (4) des Endoskops (1) verbunden ist.

11. Endoskop nach einem der Ansprüche 8 bis 10, **dadurch gekennzeichnet, dass** zwischen dem distalen Ende des Endoskops (1) und der Kappe (15) des Hygieneschutzes (2) Luft vorliegt.

## Claims

1. Hygiene protection for endoscopes having at least one working channel (14), wherein the hygiene protection (2) comprises on its distal end a cap (15) with a working channel tube (17) provided thereon and a protective sheath (21) adjacent to the cap (15), wherein the working channel tube (17) is provided at the cap (15) in a distance to the circumference of the cap (15), wherein a spraying head (18) is arranged in the cap (15), which at least proximally extends beyond the cap (15) in axial direction of the hygiene protection (2), **characterized in that** the spraying head (18) has a valve (20) at its proximal end.

2. Hygiene protection according to claim 1, **characterized in that** the spraying head (18) is arranged at the cap (15) in a distance to the circumference of the cap (15).

3. Hygiene protection according to anyone of claims 1 or 2, **characterized in that** the proximal end of the spraying head (18) extends into the distal end of a scavenging channel (13) of an endoscope (1), which is inserted into the protective sheath (21) of the hygiene protection (2), and terminates within the scavenging channel (13).

4. Hygiene protection according to anyone of claims 1 to 3, **characterized in that** a valve (28), preferably a valve with a side port (29), is attached at the proximal end of the working channel tube (17).

5. Hygiene protection according to claim 4, **characterized in that** the side port (29) is connected to a suction valve (4), which may be inserted into a valve seat (43) of an endoscope (1), which is inserted into the protective sheath (21) of the hygiene protection (2).

6. Hygiene protection according to anyone of claims 1 to 5, **characterized in that** a conical extension (34) is attached to the protective sheath (21).

7. Hygiene protection according to claim 6, **characterized in that** a valve (28) is provided at the conical extension (34) of the protective sheath (21) for connecting to the working channel tube (17).

8. Endoscope with hygiene protection, **characterized in that** the endoscope comprises a hygiene protection (2) according to any of claims 1 to 7.

9. Endoscope according to claim 8, **characterized in that** the hygiene protection (2) comprises a cap (15), which is located in the immediate vicinity of the distal end of the endoscope (1), a working channel tube (17) is guided through the working channel (14) of the endoscope and the length of the working channel tube (14) is greater than the length of the working channel (14).

10. Endoscope according to anyone of claims 8 or 9, **characterized in that** a valve (28) with a side port (29) is attached at the proximal end of the working channel tube (17) and **in that** the side port (29) is connected to the suction valve (4) of the endoscope (1).

11. Endoscope according to anyone of claims 8 to 10, **characterized in that** air is present between the distal end of the endoscope (1) and the cap (15) of the hygiene protection (2).

## Revendications

1. Protection hygiénique pour endoscope comportant au moins un canal de travail (14), la protection hygiénique (2) comprenant à son extrémité distale, une coiffe (15) comportant un tuyau de canal de travail (17) prévu sur celle-ci et une enveloppe de protection (21) adjacente à la coiffe (15), le tube de canal de travail (17) de la coiffe (15) étant prévu à distance du pourtour de la coiffe (15), une tête de pulvérisation (18) étant disposée dans la coiffe (15), s'étendant en direction axiale de la protection hygiénique (2) au moins jusqu'au le plan proximal au-dessus de la coiffe (15) et **caractérisée en ce que** la tête de pulvérisation présente une soupape (20) à son extrémité proximale.

2. Protection hygiénique selon la revendication 1, **caractérisée en ce que** la tête de pulvérisation (18) sur la coiffe (15) est disposée à distance du pourtour de la coiffe (15).

3. Protection hygiénique selon la revendication 1 ou 2, **caractérisée en ce que** la tête de pulvérisation (18) atteint avec son extrémité proximale, l'extrémité distale d'un canal de pulvérisation (13) d'un endoscope (1) introduit dans l'enveloppe de protection (21) de la protection hygiénique (2) et se termine dans le canal de pulvérisation (13).

4. Protection hygiénique selon l'une des revendications 1 à 3, **caractérisée en ce qu'**une soupape (28) est fixée à l'extrémité proximale du tube de canal de travail (17), de préférence, une soupape présentant un accès latéral (29).

5. Protection hygiénique selon la revendication 4, **caractérisée en ce que** l'accès latéral (29) est relié à une soupape d'aspiration (4) qui peut être insérée dans un siège de soupape (43) d'un endoscope (1) inséré dans l'enveloppe de protection (21) de la protection hygiénique (2).

6. Protection hygiénique selon l'une des revendications 1 à 5, **caractérisée en ce qu'**une rallonge conique (34) est fixée à l'enveloppe de protection (21).

7. Protection hygiénique selon la revendication 6, **caractérisée en ce que** sur la rallonge conique (34) de l'enveloppe de protection (21) est prévue une soupape (28) à relier avec le tube de canal de travail (17).

8. Endoscope doté d'une protection hygiénique, **caractérisé en ce que** celui-ci présente une protection hygiénique (2) selon l'une des revendications 1 à 7.

9. Endoscope selon la revendication 8, **caractérisé en ce que** la protection hygiénique (2) présente une coiffe (15) qui se situe à proximité immédiate de l'extrémité distale de l'endoscope (1), un tube de canal de travail (17) est introduit dans le canal de travail (14) de l'endoscope et la longueur du tube du canal de travail (14) est supérieure à la longueur du canal de travail (14).

10. Endoscope selon l'une des revendications 8 ou 9, **caractérisé en ce qu'**à l'extrémité proximale du tube de canal de travail (17) est fixée une soupape (28) comportant un accès latéral (29) et l'accès latéral (29) est relié à la soupape d'aspiration (4) de l'endoscope (1).

11. Endoscope selon l'une des revendications 8 à 10, **caractérisé en ce que** de l'air est présent entre l'extrémité distale de l'endoscope (1) et la coiffe (15) de la protection hygiénique (2).
